# EUROPÄISCHE PATENTSCHRIFT

(11) **EP 0 354 412 B1**
(45) Veröffentlichungstag und Bekanntmachung des Hinweises auf die Patenterteilung: **18.01.1995**
(21) Anmeldenummer: 89113827.3
(22) Anmeldetag: 27.07.1989
(51) Int. Cl.: C07D 293/12

(54) **Verfahren zur Herstellung von hochreinem Ebselen**
Process for the preparation of highly pure ebselene
Procédé de préparation d'Ebselen purifié

(30) Priorität: 10.08.1988 DE 3827093
(43) Veröffentlichungstag der Anmeldung: 14.02.1990
(73) Patentinhaber: A. Nattermann & Cie. GmbH, D-50829 Köln (DE)
(72) Erfinder: Günther, Bernd-Rainer, Dr., D-5010 Bergheim (DE); Losch, Rainer, Dr., D-5300 Bonn 1 (DE); Steiner, Klaus, Dr., D-7808 Waldkirch (DE)
(74) Vertreter: Döring, Wolfgang, Dr.-Ing. Patentanwälte Hauck, Graalfs, Wehnert, Döring, Siemons

(56) Entgegenhaltungen:
- EP-A- 0 044 971
- ACTA CHEMICA SCANDINAVICA, Band 16, Nr. 7, 1962, Seiten 1809-1810, Stockholm,SE; LARS-BÖRGE AGENÄS: "Di-(hydroxyalkyl) diselenides"
- BULLETIN DES SOCIETES CHIMIQUES BELGES, Band 75, 1966, Seiten 157-168,Bruxelles, BE; A. RUWET et al.: "Mise au point d'une synthèse du chlorured'ortho méthylsélénobenzoyle, étude de son comportement vis-a-vis des dérivésorganocadmiques"
- BERICHTE DER DEUTSCHEN CHEMISCHE GESELLSCHAFT, Band 57, Nr. 7, 9. Juli 1924,Seiten 1077-1082, Berlin, DE; R. LESSER et al.: "Über selenhaltige aromatischeVerbindungen

## Beschreibung

Die Erfindung betrifft ein Verfahren zur Herstellung von hochreinem Ebselen (2-Phenyl-1,2-benzisoselenazol-3(2H)-on) der Formel I 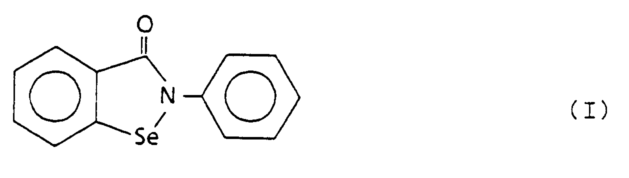
Bei Ebselen handelt es sich um eine pharmakologisch wirksame und äußerst untoxische selenorganische Verbindung mit wertvollen pharmakologischen, z.B. anti-inflammatorischen Eigenschaften (N. Dereu, E. Graf; Drugs of the Future Vol. 9, Nr. 10, 741 (1984)).

Bisher wurde Ebselen wie folgt hergestellt:
Selen wird in einer alkalisch wässrigen Lösung mit Natrium-Formaldehydsulfoxylat (Rongalit; Warenzeichen der Firma BASF) gemäß L.Tschugaeff und W. Chlopin, Ber. 47, 1269 - 1275 (1914) in alkalischer Lösung bei Temperaturen von 70 - 80^{o}C reduziert und das erhaltene Natriumdiselenid durch Abkühlen der erhaltenen Mutterlauge auskristallisiert; das erhaltene Natriumdiselenid wurde gemäß A. Ruwet und M. Renson, Bull. Soc. Chim. Belges, 75, 157-168 (1966) mit diazotierter Anthranilsäure bei einer Temperatur unterhalb 10^{o}C in wässrig-alkalischer Lösung umgesetzt, die Lösung mit Aktivkohle zur Bindung von rotem kolloidalem Selen abgeklärt, die erhaltene Lösung angesäuert und die erhaltene o-Diselenosalicylsäure abfiltriert. Sie wird in 50 bis 90%-iger Ausbeute erhalten. Die o-Diselenosalicylsäure wird sodann nach R. Lesser und R. Weiß; Ber. 46, 2640-2658 (1913) durch Erhitzen mit einem Überschuß Thionylchlorid am Rückflußkühler, Entfernen des überschüssigen Thionylchlorids und Ausziehen des erhaltenen Produktes mit Petroläther, und schließlich durch Umsetzung des so erhaltenen o-Chlorselenobenzoesäurechlorids mit Anilin in Benzol unter Eiskühlung gemäß R.Lesser und R. Weiß; Ber. 57, 1077-1082 (1924) erhalten.

Bei dem bekannten Verfahren wird aber schon in der ersten Stufe ein mit Monoselenid (2,2′-Selenobisbenzoesäure), Triselenid und amorphem Selen stark verunreinigtes, kaum absaug- oder abschleuderbares o-Diselenosalicylsäure-Produkt erhalten. Dazu macht die Umsetzung der so erhaltenen o-Diselenosalicylsäure zum o-Chlorselenobenzoesäurechlorid mit Thionylchlorid eine apparativ aufwendige Umkristallisation des giftigen und schwer zu handhabenden o-Chlorselenobenzoesäurechlorids unumgänglich. Die bisher beschriebene Darstellungsweise für Ebselen erlaubt es dazu nicht, diese Verbindung auf praktikablem Weg in genügender Menge und Reinheit herzustellen.

Da an pharmazeutische Produkte besonders hohe Reinheitsanforderungen gestellt werden, ist Aufgabe der vorliegenden Erfindung, ein Verfahren zu finden, durch das einfach, sicher und kostengünstig Ebselen mit hoher Reinheit hergestellt werden kann.

Das erfindungsgemäße Verfahren geht aus von der bekannten Reduktion von metallischem Selen mit Hilfe von Natrium-Formaldehydsulfoxylat (Rongalit; Warenzeichen der Firma BASF) in wässrig-alkalischer Lösung zu Natriumselenid, Umsetzung des erhaltenen Natriumselenids mit diazotierter Anthranilsäure in alkalischem Medium zum Natriumsalz der o-Diselenosalicylsäure, Umsetzung der freien o-Diselenosalicylsäure mit überschüssigem Thionylchlorid zum o-Chlorselenobenzoesäurechlorid und Umsetzung des Chlorselenobenzoesäurechlorids mit Anilin in einem organischen Lösungsmittel.

Das erfindungsgemäße Verfahren ist dadurch gekennzeichnet, daß
a) die Reduktion des metallischen Selens in Natronlauge mit Rongalit bei einer Temperatur zwischen 20 und 50^{o}C stattfindet und die Reaktion mit einer Nachreaktionszeit zwischen 0,5 und 2 Stunden bei gleicher Temperatur durchgeführt wird,
b) die erhaltene alkalische Natriumselenidlösung anschließend sofort mit diazotierter Anthranilsäure zwischen 0 und 10^{o}C während 1 bis 2 Stunden umgesetzt wird, die dabei anfallende alkalische o-Diselenosalicylsäurelösung mit Essigsäure auf einen pH-Wert zwischen 7,5 und 8,5 abgepuffert, die erhaltene Lösung bei einsetzender Trübung geklärt und danach mit Aktivkohle behandelt wird und dann nach Abtrennung der Aktivkohle mit Essigsäure auf einen pH-Wert zwischen 5 und 6 gebracht und die ausgefallene, abgetrennte, reine, noch wasserfeuchte o-Diselenosalicylsäure mit einem Lösungsmittel aus der Gruppe 1,2-Dichlorethan, Chloroform, Tetrachlorethan oder Petroläther, vorzugsweise mit 1,2-Dichlorethan versetzt und dieses solange cyclisch am Wasserabscheider zum Sieden erhitzt wird, bis alles Wasser azeotrop abdestilliert ist,
c) die in Stufe b) erhaltene, fein im verwendeten Lösungsmittel suspendierte o-Diselenosalicylsäure mit Thionylchlorid in Gegenwart von katalytischen Mengen Dimethylformamid umgesetzt, das Lösungsmittel und überschüssiges Thionylchlorid aus dem Reaktionsgemisch abdestilliert, gegebenenfalls vorhandenes Thionylchlorid durch mindestens noch zweimalige Zugabe des verwendeten Lösungsmittels und Abdestillieren desselben entfernt wird und
d) das in der Stufe c) angefallene gereinigte o-Chlorselenobenzoesäurechlorid in einem Lösungsmittel aus der Gruppe 1,2-Dichlorethan, Chloroform, Tetrachlorethylen und Diisopropyläther, vorzugsweise in 1,2-Dichlorethan gelöst und unter vorsichtigem Rühren die erhaltene Lösung in eine Suspension von Anilin in verdünnter Natronlauge eingebracht und das ausgefallene Reaktionsprodukt abgetrennt, neutral gewaschen und getrocknet wird.

Das getrocknete Ebselen wird vorzugsweise aus 1,2-Dichlorethan oder Butanon-2 umkristallisiert. Wasserfeuchtes Ebselen kann nach cyclischer azeotroper Destillation mit 1,2-Dichlorethan anschließend direkt aus 1,2-Dichlorethan umkristallisiert werden. Geringe Verunreinigungen des erhaltenen Reaktionsproduktes mit -SeₓS_{y}- (x = 1-8; y = 7-0) und amorphem Selen können durch eine Behandlung mit festem Natriumhydroxid und Aktivkohle in 1,2-Dichlorethan entfernt werden.

Wesentlich für das neue Verfahren zur Herstellung des Ebselens ist, daß die Reaktion in einem zweiphasigen, wäßrig-alkalischen System mit 1,2-Dichlorethan, Chloroform, Tetrachlorethylen oder Diisopropylether als organische Phase erfolgt und das in einem der oben genannten Lösungsmittel gelöste o-Chlorselenobenzoesäurechlorid während ca. 1 - 1,5 Stunden bei einer Temperatur von ca. 20 - 25^{o}C unter vorsichtigem Rühren zu einer wäßrigen Suspension von frisch destilliertem Anilin in verdünnter Natronlauge zugegeben wird. Die Auswahl des Reaktors und des Rührsystems muß so erfolgen, daß während des Rührvorganges keine Emulsionsbildung durch vertikale Rührkräfte auftreten kann. Wesentlich für das neue Verfahren ist weiter, daß nach erfolgter Umsetzung das Ebselen schnellstmöglich abgetrennt und neutralgewaschen wird.

Überraschenderweise tritt bei dieser Verfahrensweise nur eine geringe Zersetzung des Ebselens zu dem "Diselenid" der Formel IV auf. 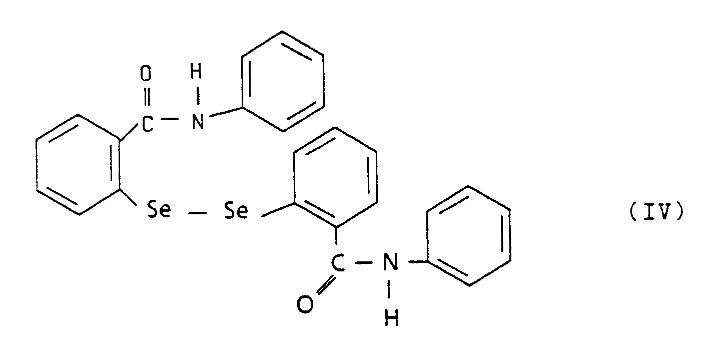
Wesentlich für das Verfahren ist weiterhin, daß das getrocknete Ebselen aus Butanon-2 oder 1,2-Dichlorethan umkristallisiert und ggfs. mit festem Natriumhydroxid und Aktivkohle in 1,2-Dichlorethan behandelt wird und das wasserfeuchte Ebselen nach zyklischer azeotroper Destillation direkt aus 1,2-Dichlorethan umkristallisiert wird. Überraschenderweise kann auf diese Weise ein hochreines Ebselen, frei von "Diselenid", -SeₓS_{y}-, und amorphem Selen erhalten werden.

Das erfindungsgemäße Herstellungsverfahren für Ebselen wird durch die folgenden Beispiele näher erläutert.

### Beispiel 1

I. In einem 180-l-Reaktor werden 12,0 kg demineralisiertes Wasser vorgelegt und anschließend 3.15 kg (40 Mol) Selen, schwarz 99,5%-ig, eingerührt, Unter Rühren werden 21,95 kg (137,2 Mol) Natronlauge 25%-ig und danach 3,43 kg (21,6 Mol) Rongalit ca. 97%-ig zugegeben. Hierbei entsteht eine dunkelbraune Lösung. Bei der Rongalitzugabe steigt die Temperatur auf 35^{o}C - 50^{o}C an. Das Reaktionsgemisch wird mit Hilfe der Mantelheizung 1 - 2 Stunden bei 45^{o}C nachgerührt.
II. In einen von außen kühlbaren Rührreaktor werden 6 kg Eis und 10,6 kg (93,2 Mol) Salzsäure 32%-ig vorgelegt. In diese Lösung werden 5,6 kg (40,0 Mol) Anthranilsäure ≧ 97%-ig eingerührt. Unter Kühlung auf 0^{o}C - 5^{o}C wird eine Lösung aus 2,9 kg (41,2 Mol) Natriumnitrit ≧ 98%-ig gelöst in 6,8 kg demineralisiertem Wasser während 1,5 - 2 Stunden zugetropft. Die Diazoniumsalzlösung muß bis zum vollständigen Verbrauch bei 0^{o}C - 5^{o}C Innentemperatur gelagert werden.
III. Die Selenidlösung aus I wird auf 10^{o}C abgekühlt. Zu dieser Lösung wird die unter II hergestellte Diazoniumsalzlösung während 1,5 - 2 Stunden unter Kühlung so zugetropft, daß die Temperatur im Reaktionsmedium immer < 10^{o}C beträgt. Der pH-Wert der Lösung muß während der gesamten Reaktionszeit ≧ 10 betragen. Nach beendeter Zugabe wird das Reaktionsgemisch ohne Kühlung 1 - 2 Stunden nachgerührt.
IV. Das Reaktionsgemisch wird unter Rühren durch vorsichtiges Zutropfen von ca. 2,5 - 3 kg (ca. 6 Mol) verdünnter Essigsäure (120 g/l) auf einen pH-Wert von 8,5 eingestellt und klarfiltriert.
V. Anschließend wird die Lösung 2 Stunden bei 20^{o}C - 25^{o}C mit 0,8 kg Aktivkohle verrührt und über einen Druckfilter klarfiltriert.
   Das Reaktionsprodukt wird unter laufender pH-Wert-Kontrolle bei Raumtemperatur (20^{o}C - 25^{o}C) mit ca. 33 kg (ca. 66 Mol) verd. Essigsäure (120 g/l) im Reaktor gefällt. Hierzu wird das Produkt bei pH 5,0 - 5,5 mittels der Essigsäure auf den obigen pH-Wert eingestellt, 30 Minuten ruhren gelassen und erneut eingestellt. Dies wird bis zu pH-Konstanz fortgeführt. Abschließend wird das Reaktionsgemisch mit ca. 3,3 kg Essigsäure ca. 98%-ig auf einen pH-Wert von 4,2 - 4,5 eingestellt. Das ausgefallene Produkt wird über eine Nutsche abgesaugt.
   1. Fällung: Ausbeute: 6,64 kg / 7,5 kg Trockensubstanz
VI. Die feuchte o-Diselenosalicylsäure wird in ca. 60 kg deminieralisiertem Wasser suspendiert. Zu dieser Suspension werden so lange ca. 5,1 kg (ca. 32 Mol) Natronlauge 25%-ig zugetropft, bis der pH-Wert 12,5 beträgt. Hierbei entsteht eine rote, trübe Lösung. Diese Lösung wird mit ca. 1 kg verd. Essigsäure (120 g/l) auf einen pH-Wert von 8,5 eingestellt. Die trübe Lösung wird filtriert. Das klare Filtrat wird mit ca. 1 kg verd. Essigsäure (120 g/l) wieder auf einen pH-Wert von 8,5 eingestellt. Die Lösung wird 1,5 Stunden mit 0,8 kg Aktivkohle verrührt. Die gekohlte Lösung wird klarfiltriert. Das Produkt wird aus der geklärten Lösung mit ca. 15 kg (ca. 30 Mol) verd. Essigsäure (120 g/l) gefällt. Das ausgefallene Produkt wird scharf abgesaugt. Der Nutschkuchen wird in ca. 32 kg Wasser, das mit Essigsäure auf einen pH-Wert von 4,5 eingestellt worden war, verrührt und anschließend wieder abgesaugt.
   2. Fällung: Ausbeute: 6,5 kg Trockensubstanz

### Beispiel 2

In einem beheizbaren 45 l Doppelmantelglasreaktor, ausgestattet mit Rührer, Innenthermometer, Destillationsbrücke mit aufsteigendem Kühler und zwischengeschaltetem T-Stück mit Hahn und gekühlter Wechselvorlage, Zulaufgefäß und Absorberturm, werden 4,5 kg (11,24 Mol) feuchte o-Diselenosalicylsäure vorgelegt und mit 17,7 kg = 14,2 l 1,2-Dichlorethan chemisch rein versetzt. Das Ganze wird unter Rühren auf Rückflußtemperatur erwärmt (Innentemperatur ca. 74^{o}C; Badtemperatur ca. 140^{o}C). Das abdestillierende Azeotrop wird in der Wechselvorlage aufgefangen, hierbei scheidet sich das ausgeschleppte Wasser ab, und das Dichlorethan zurückgeführt. Das abgeschiedene Wasser wird zurückgewogen und hierüber das Trockengewicht der o-Diselenosalicylsäure bestimmt.
Trockengewicht o-Diselenosalicylsäure = o-Diselenosalicylsäure feucht - ausgekreiste Wassermenge.

Die wasserfreie Suspension wird mit 13 ml Dimethylformamid ≧ 99%-ig versetzt. Unter Rückflußtemperatur und kräftigem Rühren werden 5,94 kg (49,45 Mol) Thionylchlorid z. Synth ≧ 99%-ig möglichst schnell zugetropft. Die Thionylchloridmenge muß der oben errechneten o-Diselenosalicylsäuremenge Trockengewicht angepaßt werden (Molverhältnis 1:4,4). Nach der Thionylchloridzugabe wird noch 1 Stunde (Ende Gasentwicklung) unter Rückfluß nachgerührt. Das 1,2-Dichlorethan wird unter Rühren abdestilliert (Badtemperatur ca. 140^{o}C); anschließend wird im Wasserringvakuum(ca. 110 mbar)getrocknet. Die zurückbleibende Schmelze wird in 7,1 l = 8,9 kg 1,2-Dichlorethan verrührt und anschließend das 1,2-Dichlorethan wie oben beschrieben abdestilliert. Dieser Vorgang wird noch zweimal wiederholt. Die zurückbleibende dunkelrote Schmelze wird mit 7,1 l = 8,9 kg 1,2-Dichlorethan aufgenommen und auf Raumtemperatur abgekühlt. Die so erhaltene Lösung wird weiter umgesetzt. Ausbeute: 5,7 kg Trockengewicht

### Beispiel 3

In einem 45 l Doppelmantelglasreaktor, ausgestattet mit einer Mantelkühlung, werden unter Kühlung 1,57 kg (39,36 Mol) Natriumhydroxid-Plätzchen in 29 kg Wasser gelöst und bei 20^{o}C mit 1,83 kg (19,68 Mol) Anilin ≧ 99% (farblos) versetzt. Unter Kühlung und kräftigem Rühren werden die ca. 10 l = 5,0 kg Trockengewicht (19,68 Mol) o-Chlorselenobenzoesäurechloridlösung in 1,2-Dichlorethan innerhalb 1 Stunde bei 20^{o}C - 25^{o}C Innentemperatur zugetropft. Das Reaktionsgemisch wird noch 1 Stunde bei 20^{o}C - 25^{o}C und einem pH-Wert von 7 - 9 nachgerührt.

Das ausgefallene Produkt wird abgesaugt, zweimal mit ca. 15 kg Wasser gewaschen und trockengesaugt. Das letzte abschließende Waschwasser muß einen pH-Wert von 7 - 7,5 haben.

Das feuchte Rohebselen wird im Vakuumtrockenschrank bei 80^{o}C und 15 mg Hg bis zur Gewichtskonstanz getrocknet. Rohebselen: Ausbeute 4,8 kg Trockengewicht Umkristallisation
aus Butanon-2:
4,3 kg getrocknetes Rohebselen werden in einem Reaktor mit 86 l Butanon-2 chemisch rein versetzt und unter Rückflußkochen und Rühren gelöst. Zu der leicht trüben Lösung werden nach Abkühlen auf 70^{o}C 0,43 kg Aktivkohle gegeben und 30 Minuten unter Rückfluß gerührt. Das klare, hellgelbe Filtrat wird unter Rühren auf 20^{o}C abgekühlt. Der Niederschlag wird abgesaugt und im Vakuumtrockenschrank bei 60^{o}C und 15 mm Hg bis zur Gewichtskonstanz getrocknet.
Ausbeute: 3,0 kg Trockengewicht - Fp: 181 - 183^{o}C

Aus 1,2-Dichlorethan:
4,3 kg Trockengewicht wasserfeuchtes Rohebselen werden in einem beheizbaren Reaktor in 43 l = 53,75 kg 1,2-Dichlorethan chemisch rein und unter Rühren auf Rückflußtemperatur erhitzt und durch azeotrope Destillation getrocknet. Während der azeotropen Destillation entsteht eine fast klare Lösung, die mit einer Suspension von 0,43 kg Aktivkohle in 1,2-Dichlorethan versetzt wird. Das Ganze wird 30 Minuten unter Rückfluß gerührt und anschließend klarfiltriert. Das klare, hellgelbe Filtrat wird unter Rühren auf ca. 20^{o}C abgekühlt. Der ausgefallene Niederschlag wird abgesaugt und bei 60^{o}C und 15 mm Hg bis zur Gewichtskonstanz getrocknet.
Ausbeute: 3,27 kg Trockengewicht - Fp: 181 - 183^{o}C

### Beispiel 4

Behandlung von Ebselen mit festem Natriumhydroxid, Aktivkohle in 1,2-Dichlorethan.

In dem 250 l VA-Reaktor werden bei Raumtemperatur 20,0 kg Ebselen, 200 l 1,2-Dichlorethan rein, 1,0 kg Natriumhydroxid, granuliert, Merck N. 6467, und 2 kg Aktivkohle vorgelegt und unter Rühren 2 Stunden unter Rückfluß gekocht. Die heiße Lösung wird bei ca. 80^{o}C - 90^{o}C klarfiltriert. Das klare, hellgelbe Filtrat wird unter Rühren auf 20^{o}C abgekühlt. Das Kristallisat wird abgesaugt. Der Nutschkuchen wird mit 5 - 7 l 1,2-Dichlorethan, rein, nachgewaschen und bei 60^{o}C - 70^{o}C und 20 - 100 mbar getrocknet.
Ausbeute: 17 kg (- 85% d. Th.).

## Patentansprüche

1. Verfahren zur Herstellung von 2-Phenyl-1,2-benzisoselenazol-3(2H)-on (Ebselen) durch Reduktion von metallischem Selen mit Hilfe von Natrium-Formaldehydsulfoxylat (Rongalit; Warenzeichen der Firma BASF) in wässrig-alkalischer Lösung zu Natriumselenid, Umsetzung des erhaltenen Natriumselenids mit diazotierter Anthranilsäure in alkalischem Medium zum Natriumsalz der o-Diselenosalicylsäure, Umsetzung der freien o-Diselenosalicylsäure mit überschüssigem Thionylchlorid zum o-Chlorselenobenzoesäurechlorid und Umsetzung des Chlorselenobenzoesäurechlorids mit Anilin in einem organischen Lösungsmittel, dadurch gekennzeichnet, daß
a) die Reduktion des metallischen Selens in Natronlauge mit Rongalit bei einer Temperatur zwischen 20 und 50^{o}C stattfindet und die Reaktion mit einer Nachreaktionszeit zwischen 0,5 und 2 Stunden bei gleicher Temperatur durchgeführt wird,
b) die erhaltene alkalische Natriumselenidlösung anschließend sofort mit diazotierter Anthranilsäure zwischen 0 und 10^{o}C während 1 bis 2 Stunden umgesetzt wird, die dabei anfallende alkalische o-Diselenosalicylsäurelösung mit Essigsäure auf einen pH-Wert zwischen 7,5 und 8,5 abgepuffert, die erhaltene Lösung bei einsetzender Trübung geklärt und danach mit Aktivkohle behandelt wird und dann nach Abtrennung der Aktivkohle mit Essigsäure auf einen pH-Wert zwischen 5 und 6 gebracht und die ausgefallene, abgetrennte, reine, noch wasserfeuchte o-Diselenosalicylsäure mit einem Lösungsmittel aus der Gruppe 1,2-Dichlorethan, Chloroform, Tetrachlorethan oder Petroläther, vorzugsweise mit 1,2-Dichlorethan versetzt und dieses solange cyclisch am Wasserabscheider zum Sieden erhitzt wird, bis alles Wasser azeotrop abdestilliert ist,
c) die in Stufe b) erhaltene, fein im verwendeten Lösungsmittel suspendierte o-Diselenosalicylsäure mit Thionylchlorid in Gegenwart von katalytischen Mengen Dimethylformamid umgesetzt, das Lösungsmittel und überschüssiges Thionylchlorid aus dem Reaktionsgemisch abdestilliert, gegebenenfalls vorhandenes Thionylchlorid durch mindestens noch zweimalige Zugabe des verwendeten Lösungsmittels und Abdestillieren desselben entfernt wird und
d) das in der Stufe c) angefallene gereinigte o-Chlorselenobenzoesäurechlorid in einem Lösungsmittel aus der Gruppe 1,2-Dichlorethan, Chloroform, Tetrachlorethylen und Diisopropyläther, vorzugsweise in 1,2-Dichlorethan gelöst und unter vorsichtigem Rühren die erhaltene Lösung in eine Suspension von Anilin in verdünnter Natronlauge eingebracht und das ausgefallene Reaktionsprodukt abgetrennt, neutral gewaschen und getrocknet wird.

2. Verfahren gemaß Patentanspruch 1, dadurch gekennzeichnet, daß das getrocknete Ebselen aus 1,2-Dichlorethan oder Butanon-2 umkristallisiert wird.

3. Verfahren gemäß Patentanspruch 1, dadurch gekennzeichnet, daß das in der Stufe d) anfallende wasserfeuchte Ebselen nach Entfernung von Wasser durch cyclische azeotrope Destillation mit 1,2-Dichlorethan anschließend direkt aus 1,2-Dichlorethan umkristallisiert wird.

## Claims

1. A process for the production of 2-phenyl-1,2-benzisoselenazol-3(2H)-one (ebselen) by reduction of metallic selenium by means of sodium formaldehyde sulfoxylate (Rongalit; trade mark of the company BASF) in aqueous alkaline medium to yield sodium selenide, subjecting the resulting sodium selenide to reaction with diazotized anthranilic acid in alkaline reaction medium to yield the sodium salt of o-diselenosalicylic acid, subjecting to reaction the free o-diselenosalicylic acid with a excess of thionyl chloride to yield o-chloroselenobenzoic acid chloride and subjecting to reaction the resulting chloroselenobenzoic acid chloride with aniline in an organic solvent, wherein
a) the reduction of the metallic selenium in soda lye with Rongalit is effected at a temperature between 20 and 50 °C and the reaction mixture, after having added all reaction components, is kept at the applied reaction temperature for another 0.5 to 2 hours,
b) the resulting alkaline solution of sodium selenide is subjected immediately thereafter to reaction with diazotized anthranilic acid at a temperature between 0 and 10 °C for 1 to 2 hours; the resulting alkaline solution of o-diselenosalicylic acid is buffered with acetic acid to a pH between 7.5 and 8.5, the thus resulting solution is clarified as soon as it becomes turbid and thereafter is treated with activated charcoal and after separation of the charcoal, the solution is mixed with acetic acid to a pH value between 5 and 6, and the precipitated separated pure o-diselenosalicylic acid still moist with water is mixed with a solvent selected from the group of 1,2-dichloroethane, chloroform, tetrachloroethane or petrol ether, preferably with 1,2-dichloroethane, and is heated to boiling with a cyclic water separator for such a time, until all of the water has been distilled off azeotropically,
c) the o-diselenosalicylic acid obtained in step b) and suspended in the used solvent in finely divided form is subjected to reaction with thionyl chloride in the presence of catalytic amounts of dimethylformamide; the solvent and the excess thionyl chloride is distilled off from the reaction mixture; possibly still present thionyl chloride is removed by adding at least twice the used solvent and distilling it off and
d) the purified o-chloroselenobenzoic acid chloride obtained in step c) is dissolved in a solvent selected from the group consisting of 1,2-dichloroethane, chloroform, tetrachloroethylene and diisopropylether, preferably in 1,2-dichloroethane, and, with careful stirring, the resulting solution is added to a suspension of aniline in diluted soda lye; and the precipitated reaction product is separated, washed until neutral and dried.

2. The process according to claim 1, wherein the dried ebselen is recrystallized from 1,2-dichloroethane or butanone-2.

3. The process according to claim 1, wherein the ebselen product moist of water obtained in step d) is recrystallized directly from 1,2-dichloroethane after removal of the water by cyclic azeotropic distillation with 1,2-dichloroethane.

## Revendications

1. Procédé de préparation de la 2-phényl-1,2-benzisosélénazole-3(2H)-one (Ebselen) par la réduction du sélénium métallique à l'aide de formaldéhydesulfoxylate de sodium (Rongalit : marque de fabrique de la firme BASF) en solution aqueuse-alcaline, en séléniure de sodium, conversion du séléniure de sodium obtenu à l'aide d'acide anthranilique diazoté en milieu alcalin, en sel de sodium de l'acide o-disélénosalicylique, conversion de l'acide o-disélénosalicylique avec du chlorure de thionyle en excès en chlorure d'acide o-chlorosélénobenzoïque et réaction du chlorure d'acide chlorosélénobenzoïque avec l'aniline dans un solvant organique, caractérisé en ce que
a) on entreprend la réduction du sélénium métallique en lessive alcaline avec le Rongalit à une température comprise entre 20 et 50°C et on réalise la réaction avec une durée de postréaction comprise entre 0,5 et 2 heures à même température,
b) on convertit ensuite immédiatement la solution de séléniure de sodium alcaline obtenue avec de l'acide anthranilique diazoté entre 0 et 10°C pendant une à 2 heures, on tamponne la solution d'acide o-disélénosalicylique alcaline ainsi obtenue avec de l'acide acétique jusqu'à une valeur de pH comprise entre 7,5 et 8,5, on clarifie la solution obtenue en cas de turbidité commençante et on la traite ensuite par du charbon activé et, après séparation du carbone activé, on amène alors le pH avec de l'acide acétique à une valeur comprise entre 5 et 6 et on ajoute ensuite à l'acide o-disélénosalicylique précipité, séparé, pur, encore humide d'eau, un solvant choisi dans le groupe formé par le 1,2-dichloréthane, le chloroforme, le tétrachloréthane, ou l'éther de pétrole, de préférence le 1,2-dichloréthane et on chauffe ensuite l'ensemble ainsi obtenu de manière cyclique au séparateur d'eau jusqu'à l'ébullition jusqu'à ce que la totalité de l'eau soit éliminée par distillation azéotropique,
c) on fait réagir l'acide finement suspendu dans le solvant employé, obtenu à l'étape b), avec le chlorure de thionyle en présence de quantités catalytiques de diméthylformamide, on élimine le solvant et le chlorure de thionyle excédentaire du mélange réactionnel par distillation, on chasse le chlorure de thionyle éventuellement présent par une addition au moins double du solvant employé et élimination par distillation de celui-ci et
d) on dissout le chlorure d'acide o-chlorosélénobenzoïque dans un solvant choisi dans le groupe formé par le 1,2-dichloréthane, le chloroforme, le tétrachloréthylène et l'éther diisopropylique, de préférence dans le 1,2-dichloréthane et on introduit la solution obtenue, sous agitation précautionneuse, dans une suspension d'aniline dans une lessive sodique diluée et on sépare le produit de la réaction précipité, on le lave jusqu'à neutralité et on le sèche.

2. Procédé suivant la revendication 1, caractérisé en ce que l'on recristallise l'Ebselen séché dans le 1,2-dichloréthane, ou la butanone-2.

3. Procédé suivant la revendication 1, caractérisé en ce qu'après élimination de l'eau par distillation cyclique azéotropique avec le 1,2-dichloréthane, on recristallise ensuite directement l'Ebselen présent humide d'eau dans le 1,2-dichloréthane.
